# EUROPEAN PATENT SPECIFICATION

(11) **EP 4 103 124 B1**
(45) Date of publication and mention of the grant of the patent: **10.12.2025**
(21) Application number: 21705135.8
(22) Date of filing: 10.02.2021
(51) Int. Cl.: A61F 13/66, A61L 15/40, A61L 15/42, A61L 15/46

(54) **ABSORBENT UNDERGARMENT**
ABSORBIERENDES UNTERBEKLEIDUNGSSTÜCK
SOUS-VÊTEMENT ABSORBANT

(30) Priority: 11.02.2020 WO PCT/SE2020/050141
(43) Date of publication of application: 21.12.2022
(73) Proprietor: Essity Hygiene and Health Aktiebolag, 405 03 Göteborg (SE)
(72) Inventor: HUSMARK, Ulrika, 405 03 GÖTEBORG (SE); RYTTSÉN, Frida, 405 03 GÖTEBORG (SE)
(74) Representative: Hoffmann Eitle
(86) International application number: PCT/EP2021/053118
(87) International publication number: WO 2021/160627

(56) References cited:
- JP-A- 2013 112 903
- JP-A- 2016 079 530
- US-A1- 2014 039 432

## Description

### TECHNICAL FIELD

The present disclosure relates to an absorbent undergarment for the purpose of absorbing body fluids such as urine and menstrual fluid.

The present disclosure further relates to a method for the manufacture of an absorbent undergarment of the above-mentioned type.

### BACKGROUND

Absorbent undergarments are worn against the skin or near the crotch region for the purpose of absorbing body fluids such as urine and menstrual fluid. Conventionally, such absorbent undergarments comprise a fabric layer and an absorbent pad having absorbent properties.

However, the entrapment of body liquids in the absorbent pad of the absorbent undergarment may result in unwanted side-effects such as microbial growth, odors and disturbance of the natural skin pH. These factors may in turn cause different forms of skin irritation and primary or secondary skin infections. For example, an increase in skin pH may result in an increased activity of enzymes such as lipases and proteases. Further, the development of odors causes discomfort and is a source of embarrassment for the user.

The use of silver antimicrobial fibers in absorbent undergarments to control odor and microbial growth has been previously known in the art by, for example international patent application PCT/CA2014/000389. However, silver is of limited supply and the methods for obtaining and processing silver has a negative environmental impact. Anti-microbial coatings are also disclosed in US 2014/039432 A1 wherein the cotton is also treated with a hydrophilic composition such as poly(acrylic acid).

Thus, there is still a need in the art for improved absorbent undergarments having anti-odor and skin-beneficial effects.

### SUMMARY

The present inventors have found that by providing an absorbent undergarment according to claim 1 and a method according to claim 9, the above objectives are at least partially met.

Therefore, according to one aspect, the present invention relates to an absorbent undergarment comprising a fabric layer defining a waist opening, two leg openings and a crotch region between the leg openings. The absorbent undergarment further comprises an absorbent pad covering at least part of the crotch region and is permanently attached to the fabric layer. The absorbent pad comprises at least one absorbent layer and a skin-facing layer, wherein at least one layer, selected from the absorbent layer, the skin-facing layer or an optionally present wicking layer, comprises naturally-derived fibers. Further, the naturally-derived fibers comprise a polycarboxylic acid. The polycarboxylic acid has at least one carboxyl group which reacts with the free hydroxyl group of the naturally-derived fibers to form a covalent bond. The polycarboxylic acid has at least one additional free carboxyl group that is not bonded to the naturally-derived fibers. The free carboxyl group confers a low pH to the treated layer resulting in anti-microbial effects that remain present after laundering of the absorbent undergarment. Further, the polycarboxylic acid is a natural and sustainable additive.

The polycarboxylic acid may have between two to ten carboxyl groups. A first carboxyl group is required to allow binding of the polycarboxylic acid to the material constituting the absorbent undergarment, whereas the additional carboxyl groups (two to ten) provide the acidic properties. Preferably, the polycarboxylic acid has three to five carboxyl group, and most preferably three carboxyl groups.

The polycarboxylic acid may have 2 to 12 carbon atoms in a main chain, e.g., 4 to 6 carbon atoms in a main chain, wherein the polycarboxylic acid is preferably selected from the group consisting of citric acid, isocitric acid, citraconic acid, tartaric acid, itaconic acid, 1,2,3-propanetricarboxylic acid, 1,2,3,4-butanetetracarboxylic acid, glutaric acid, alpha-ketoglutaric acid, malic acid, malonic acid, 2-hydroxymalonic acid, oxalic acid, oxalosuccinic acid, succinic acid, carboxy succinic acid, 1,2 dimethylsuccinic acid, adipic acid, pimelic acid, 2-methyl tricarballylic acid, aconitic acid, 1,2,4-butane tricarboxylic acid, polymers of acrylic acid, and mixtures thereof, wherein the acid is preferably citric acid. Use of succinic acid, citric acid, and α-ketoglutaric acid as an additive has been shown to be safe in use in the food industry. Further, the carboxylgroups in citric acid are in close vicinity to each other.

The at least one carboxyl group in the polycarboxylic acid may be covalently bound to the naturally-derived fibers by forming an ester bond with a hydroxyl group present in the naturally-derived fibers. An ester bond is a covalent bond thus allowing the polycarboxylic acid to chemically bond to the fibers in the material of the absorbent undergarment.

The selected layer may comprise between 20% to 100% naturally-derived fibers, alternatively between 40% and 100% naturally-derived fibers or between 60% and 100% naturally-derived fibers.

The naturally-derived fibers may be selected from the group consisting of natural fibers and man-made regenerated fibers from a natural source. No chemical processes are required for the production of natural fibers. On the other hand, the manufacturing process of man-made regenerated fibers is such that it allows for the modification of the structure and properties of the man-made regenerated fibers.

The naturally-derived fibers may be selected from the group consisting of cotton, wool, silk, cellulose, regenerated cellulose, rayon, viscose, modal, lyocell, tencel, bamboo, hemp, flax, ramie, coir or banana. The naturally-derived may be selected from the group consisting of cotton or viscose fibers. The production of cotton fibers does not involve the (partial) degradation of cellulose. However, the cost of manufacturing is generally lower for viscose fibers.

The polycarboxylic acid in the present disclosure may be bound to naturally-derived fibers through an ester bond. An ester bond is a covalent bond thus allowing the polycarboxylic acid to chemically bond to the fibers in the material of the absorbent undergarment. A catalyst may be used to accelerate the esterification reaction. The catalyst may be selected from the group consisting of sodium hypophosphite, disodium monohydrogen phosphate, sodium dihydrogen phosphate, sodium salt and maleic acid or sodium pyrophosphate. Further, a curing step may be included.

The selected layer comprising the polycarboxylic acid according to the present disclosure, may be substantially free of petroleum-based materials. Petroleum is a non-renewable resource and the supply thereof is therefore limited.

The treated layer comprising the acidic solution according to the present disclosure may have a pH after washing of from 2.5 to 6.5 when measured according to the method described herein. The low pH inhibits microbial growth within the absorbent undergarment and contributes to an acidic environment between the absorbent undergarment and the skin in the crotch region. Studies have shown that natural skin surface pH is on average below 5, which is beneficial for its resident flora, see e.g. Lambers H et al (2006), Int J Cosmet Sci, 28 (5), 359-70.

The present disclosure further pertains to a method for manufacturing an absorbent undergarment as defined in this disclosure, comprising a step of treating naturally-derived fibers with a treatment solution comprising one or more polycarboxylic acid, or optionally a mixture of one or more polycarboxylic acids with salts thereof. The polycarboxylic acid may have two to ten carboxyl groups, preferably three to five carboxyl groups, and more preferably three carboxyl groups. The polycarboxylic acid may have 2 to 12 carbon atoms in a main chain, e.g., 4 to 6 carbon atoms in a main chain, wherein the compound is preferably selected from the group consisting of citric acid, isocitric acid, citraconic acid, tartaric acid, itaconic acid, 1,2,3-propanetricarboxylic acid, 1,2,3,4-butanetetracarboxylic acid, glutaric acid, alpha-ketoglutaric acid, malic acid, malonic acid, 2-hydroxymalonic acid, oxalic acid, oxalosuccinic acid, succinic acid, carboxy succinic acid, 1,2 dimethylsuccinic acid, adipic acid, pimelic acid, 2-methyl tricarballylic acid, aconitic acid, 1,2,4-butane tricarboxylic acid, polymers of acrylic acid, and mixtures thereof, wherein the acid is preferably citric acid. Optionally, the absorbent undergarment is dried after the treatment step. Finally, the absorbent garment is cured.

At least condition (iv), preferably all, of the following conditions is fulfilled during manufacturing of the absorbent underwear:
(i) the concentration of the polycarboxylic acid in the treatment solution is 2 to 80 wt.-%, preferably 2.5 to 50 wt.-% and more preferably 10 to 20% wt.-% based on the total weight of the treatment solution,
(ii) the treatment time is 20 sec to 30 min, preferably 30 sec to 15 min, in particular 30 sec to 5 min,
(iii) the curing time is 10 sec to 10 min, preferably 10 sec to 1 min, in particular 10 sec to 30 sec,
(iv) the curing temperature is 140 °C to 200 °C, preferably 140 °C to 180 °C, more preferably 140 °C to 170 °C.

The treatment solution may comprise a catalyst. The catalyst may be selected from the group consisting of sodium hypophosphite, disodium monohydrogen phosphate, sodium dihydrogen phosphate, sodium salt of maleic acid or sodium pyrophosphate.

The selected layer of the absorbent undergarment according to the present disclosure may have a pH after one washing cycle of from 2.5 to 6.5, preferably from 4.6 to 5.7, more preferably from 4.6 to 5.2, when measured according to the method described herein. The selected layer of the absorbent undergarment according to the present disclosure may have a pH after 5 washing cycles of from 2.5 to 6.5 when measured according to the method described herein.

### BRIEF DESCRIPTION OF THE FIGURES

The present disclosure will be further explained hereinafter by means of examples and with reference to the appended drawings wherein:
- Fig. 1: shows a graph depicting bacterial growth on cotton fabric;
- Fig. 2: shows a graph depicting bacterial growth on fabric consisting of a bamboo viscose/cotton blend;
- Fig. 3: shows a front view of the absorbent undergarment;
- Fig. 4: shows a cross-sectional view of a portion of the absorbent undergarment;
- Fig. 5: shows a cross-sectional view of a portion of the absorbent undergarment according to an alternative embodiment;

### DETAILED DESCRIPTION

The present disclosure relates to an absorbent undergarment comprising a fabric layer defining a waist opening, two leg openings and a crotch region between the leg opening. The absorbent undergarment further comprises an absorbent pad covering at least part of the crotch region and is permanently attached to the fabric layer. The absorbent pad comprises at least one absorbent layer and a skin-facing layer. Optionally, the absorbent pad may comprise a wicking layer.

In the absorbent undergarment, at least one layer selected from said skin-facing layer or said at least one absorbent layer, and/or an optionally present wicking layer comprises naturally-derived fibers comprising a polycarboxylic acid. The polycarboxylic acid may have between two to ten carboxyl groups, preferably three to five and more preferably three carboxyl groups. At least one carboxyl group is attached to the fibers by an esterification process forming an ester bonding between at least one free carboxyl group of the polycarboxylic acid and at least one free hydroxyl group of the fibers.

As used herein, the term "naturally-derived fibers" refers to fibers derived from a natural source. Naturally-derived fibers include natural fibers which may be plant-based such as cotton; or animal-based such as wool or silk. Naturally-derived fibers further include man-made regenerated fibers from a natural source such as regenerated cellulose, rayon, viscose, modal, lyocell, tencel, bamboo, hemp, flax, ramie, coir and banana. Synthetic man-made fibers such as nylon, acrylic, polyester or elastane, are not considered to be naturally-derived fibers according to the present disclosure.

As used herein, the term "absorbent undergarment" refers to garments that are worn and intended to be placed against the skin of the wearer to absorb and contain body exudates, such as urine and menstrual fluid. The undergarment according to the present disclosure is intended to be laundered or otherwise restored after use for reuse as a sanitary article.

By "laundering" it is meant that the absorbent undergarment can be cleaned by laundering. The absorbent undergarment may thus be subjected to an aqueous solution containing detergent without losing its structural features. This aqueous solution may be heated as part of the laundering process, e.g. to 30°C or 60°C.

The term "fabric" as used in the present disclosure may refer to a single or multiple layers of fabrics. The fabric may be knitted or woven.

By "permanently attached", it is meant that the absorbent pad is not intended to separate from the fabric layer before, during, or after use. The absorbent pad is not necessarily directly bonded to the fabric layer, but instead may be attached via the leg opening seams.

As used herein, the term "acidic solution" refers to a solution having an acid pH, i.e. less than pH 7.

In all figures in the following detailed description, the same reference numerals will be used to indicate the same elements.

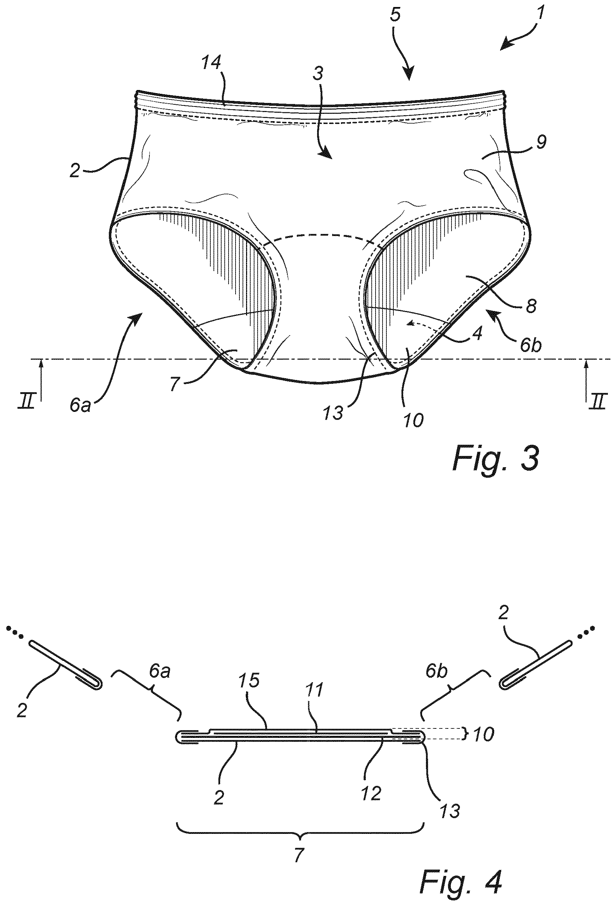

Figure 3 is a front view of the absorbent undergarment according to the present disclosure. The absorbent undergarment 1 comprises a fabric layer 2 having a front side 3, a back side 4 and a waist opening 5. The fabric layer 2 has two leg openings 6a and 6b, and a crotch region 7 in between the leg openings 6a and 6b. The fabric layer 2 further includes an interior surface 8 (i.e., a surface of the fabric layer 2 that wholly or partially contacts the body of the wearer) and an exterior surface 9 (i.e. a surface of the fabric layer 2 that does not contact the body of the wearer). The absorbent undergarment 1 further comprises an absorbent pad 10 covering at least part of said crotch region. In figure 3, a leg opening seam 13 lines the leg openings 6a and 6b. The leg opening seam 13 may be provided by conventional stitching or gluing techniques. The leg opening seam may comprise an elastic member such as elastic bonding film. Further, the leg opening seam may serve to bond the skin-facing layer, the absorbent pad, the outer layer and the optionally present wicking layer together. The absorbent undergarment 1 may further comprise a waist band 14.

Figure 4 shows a cross-sectional view of the crotch region of the absorbent undergarment 1. The absorbent undergarment according to the present disclosure comprises an absorbent structure, the absorbent pad 10, arranged on the interior surface 8 of the fabric layer 2 and covering a least part of the crotch region 7. The absorbent pad 10 may comprise 1 or more absorbent layers 11. In figure 4, only 1 absorbent layer is shown to increase clarity of the figure. Further, the absorbent pad 10 comprises a liquid-impermeable barrier 12 located between the one or more absorbent layers 11 and the fabric layer 2. The liquid-impermeable barrier layer 12 prevents liquid from leaking through the one or more absorbent layers 11 into the fabric layer 2. The absorbent pad further comprises a soft skin-facing layer 15 facing the skin-side of the user. The different layers of the absorbent pad 10, but additionally also the fabric layer 2, are bonded together via the leg opening seam 13.

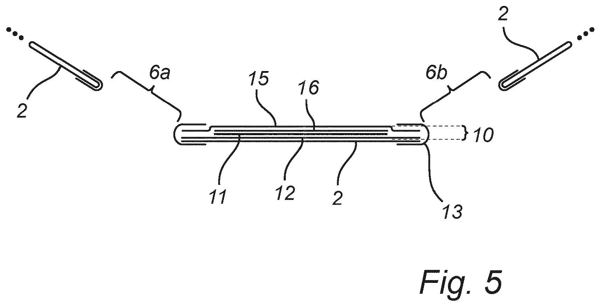

Figure 5 shows an alternative embodiment of the absorbent undergarment according to this disclosure wherein the absorbent pad further comprises a wicking layer 16 underneath the skin-facing layer 15.

The skin-facing layer 15 comprises a water-permeable material thus allowing the body fluids to migrate to the underlying absorbent layer or layers. A skin-facing layer having an open structure may further reduce the wetting zone leading to a prolonged skin beneficial effect when used in an absorbent undergarment in accordance with the present disclosure. The skin-facing layer 15 may be constructed of any suitable fabric, including naturally-derived fibers selected from the group consisting of cotton, wool, silk, cellulose, regenerated cellulose, rayon, viscose, modal, lyocell, tencel, bamboo, hemp, flax, ramie, coir or banana. Alternatively, the skin-facing layer 15 may be constructed of synthetic fibers selected from the group consisting of nylon, acrylic, polyester or elastane. Further, the skin-facing layer 15 may be constructed of a blend or a mixture of naturally-derived and/or synthetic fibers. The materials used for construction of the skin-facing layer 15 should be soft and non-irritating to the skin and be readily penetrated by body fluid, such as urine or menstrual fluid. According to the present disclosure, the skin-facing layer 15 should be launderable. When said polycarboxylic acid is provided to the skin-facing layer, the skin-facing layer comprises naturally-derived fibers comprising a free hydroxyl group to enable chemical bonding of the polycarboxylic acid to the skin-facing layer. Thus, the skin-facing layer may comprise between 20% and 100% naturally-derived fibers, more preferably between 40% and 100% naturally-derived fibers and most preferably between 60% and 100% naturally-derived fibers.

The skin-facing layer 15 may have wicking features to allow the moisture to spread away from the wearer and into the absorbent layer or layers. Further, wicking enables an efficient spread of the moisture therefore allowing the moisture to be received in a wider area of the underlying one or more absorbent layers 11. This feature is particularly relevant for users suffering from stress incontinence as spurts of urine may cause the absorbent layer to be saturated rapidly at the point of impact. By spreading the volume of the fluid over a wider receiving area in the absorbent layer, the wicking features of the skin-facing layer 15 increase the overall absorptive capacity of the absorbent layer 11. As shown in figure 5, a separate wicking layer 16 may be provided underneath the skin-facing layer 15.

The wicking layer 16 may comprise a water-permeable material thus allowing the body fluids to migrate to the underlying absorbent layer or layers. The wicking layer 16 may be constructed of any suitable fabric, including naturally-derived fibers selected from the group consisting of cotton, wool, silk, cellulose, regenerated cellulose, rayon, viscose, modal, lyocell, tencel, bamboo, hemp, flax, ramie, coir or banana. Alternatively, the wicking layer 16 may be constructed of synthetic fibers selected from the group consisting of nylon, acrylic, polyester or elastane. Further, the wicking layer 16 may be constructed of a blend or a mixture of naturally-derived and/or synthetic fibers. According to the present disclosure, the wicking layer 16 should be launderable. When said polycarboxylic acid is provided to the wicking layer, the wicking layer comprises naturally-derived fibers comprising a free hydroxyl group to enable chemical bonding of the polycarboxylic acid to the wicking layer. Thus, the wicking layer may comprise between 20% and 100% naturally-derived fibers, more preferably between 40% and 100% naturally-derived fibers and most preferably between 60% and 100% naturally-derived fibers.

The absorbent pad 10 for use in an absorbent undergarment may contain one or multiple absorbent layers 11, capable of absorbing liquid and releasing the liquid during laundering. Release of the absorbed liquid may be beneficial as it enables the absorbent undergarment to be restored for reuse. Reusable absorbent undergarments provide a more sustainable alternative to the commonly-used disposable hygiene articles that are not intended to be reused.

The absorbent layer 11 may comprise any material capable of absorbing fluid, such as woven or nonwoven microfiber or polymer knits, fabric formed from hydrophilic fibers, absorbent fibers or powders. Alternatively, the absorbent layer may comprise a material treated with a hydrophilic treatment. The absorbent layer may also comprise a material having an open cell porous structures such as high loft or synthetic fibers having reservoir properties. When said polycarboxylic acid is provided to the absorbent layer, the absorbent layer comprises naturally-derived fibers comprising a free hydroxyl group to enable chemical bonding of the polycarboxylic acid to the absorbent layer. Thus, the absorbent layer may comprise between 20% and 100% naturally-derived fibers, more preferably between 40% and 100% naturally-derived fibers and most preferably between 60% and 100% naturally-derived fibers.

The liquid-impermeable barrier layer 12 may comprise any suitable material or combinations of material that prevents liquid from migrating from the absorbent pad to the fabric layer. The liquid-impermeable barrier layer 12 may comprise a hydrophobic woven or nonwoven material having inherent hydrophobic properties, or that has been treated to become hydrophobic. Examples of hydrophobic materials for treating the barrier layer are polymers such as silicone, polyurethane and combinations thereof. The liquid-impermeable barrier layer 12 may comprise a microporous polymer film, e.g. a polyethylene, PTFE or polyurethane film, or combinations thereof. Laminates of polymer films and nonwoven materials may also be used. The liquid-impermeable barrier layer 12 may comprise a moisture-impermeable polymer layer such as urethane wax on the surface facing away from the wearer.

The liquid-impermeable barrier layer 12 is preferably breathable, to allow vapor to escape from the absorbent undergarment, while preventing liquid from passing through the fabric layer. Further, the liquid-impermeable barrier layer 12 may be constructed of an elastic material, thus providing the absorbent pad 10 with the required flexibility to adapt to the user's anatomy and movements.

This improved fit increases the wearer's comfort during use and helps to prevent leakage from migrating through the undergarment's leg openings.

The fabric layer 2 may be constructed of any suitable fabric, including naturally-derived fibers selected from the group consisting of cotton, wool, silk, cellulose, regenerated cellulose, rayon, viscose, modal, lyocell, tencel, bamboo, hemp, flax, ramie, coir or banana. Alternatively, the fabric layer 2 may be constructed of synthetic fibers selected from the group consisting of nylon, acrylic, polyester or elastane. Further, the fabric layer 2 may be constructed of a blend or a mixture of naturally-derived and/or synthetic fibers. The fabric layer may comprise a stretchable fabric, e.g. spandex, so that the absorbent undergarment can provide a firm fit while at the same time adapting to the wearer's movements thus preventing any leakage from migrating through the leg openings and keeping the absorbent pad in place. The fabric layer 2 is preferably breathable to allow vapor to escape from the wearer's skin and from the absorbent structure. The fabric layer 2 may be constructed of the same material as the skin-facing layer to give the absorbent underwear a more uniform appearance and to make the absorbent pad more discrete.

The skin-facing layer 15, the absorbent pad 10, the fabric layer 2 and the optionally present wicking layer 16 may be bonded by conventional stitching, gluing techniques or via a bonding film such as tape.

According to the present disclosure, it is beneficial to provide the absorbent undergarment with an acidic pH that will be maintained after laundering the absorbent undergarment, i.e. a permanent acidification. The acidic pH reduces microbial growth thus increasing the hygienic properties of the absorbent undergarment while at the same time preventing unwanted odors caused by microbial metabolites. This is achieved by providing a polycarboxylic acid to the absorbent undergarment. Polycarboxylic acids are organic and biodegradable and therefore have a limited environmental impact.

The permanent acidification may be achieved by providing an acidic treatment comprising a polycarboxylic acid comprising two to ten carboxyl groups. A first carboxyl group is required to allow binding of the polycarboxylic acid to the material constituting the absorbent undergarment, whereas the additional carboxyl groups provide the acidic properties. The polycarboxylic acid binds through the formation of an esterbond with a free hydroxyl group in the material of the absorbent article. In this respect, it is essential that the material to which the acidic solution is to be applied, comprises naturally-derived fibers as these fibers contain a free hydroxyl group.

The polycarboxylic acid may comprise at least three carboxyl groups, such as citric acid. After binding of the carboxyl group to the fibers in the absorbent undergarment, two carboxyl groups remain to provide the absorbent undergarment with the desired acidic pH. The presence of several remaining carboxyl groups after binding, results in a further lowering of the pH of the absorbent undergarment.

The acidic solution may comprise between 2 and 80% polycarboxylic acid, more preferably between 5 and 50% polycarboxylic acid and most preferably between 10 and 30% polycarboxylic acid. The acidic solution may be applied to a layer selected from the absorbent layer, the skin-facing layer, and/or optionally present wicking layer, or several selected layers, for a time period between 20 seconds to 30 minutes, more preferably between 30 second and 15 minutes and most preferably between 30 seconds and 5 minutes. A subsequent curing step enables the carboxylgroup of the polycarboxylic acid to bond with the hydroxyl group in the naturally-derived fiber. The treatment solution may comprise a catalyst to accelerate the process.

The acidic solution may be essentially homogenously applied to a layer selected from the absorbent layer, the skin-facing layer, and/or optionally present wicking layer. The acidic solution may be applied in a patterned manner, covering only part of the surface of the layer on which it is applied. It is contemplated that in one and the same product, the acidic solution may be homogenously applied to one selected layer, and furthermore applied in a patterned manner on another selected layer.

The present disclosure further pertains to a method for manufacturing an absorbent undergarment as disclosed in the above paragraphs. The method comprises the steps of disposing an absorbent pad comprising at least one absorbent layer and a skin facing layer, on a fabric layer. Next, the naturally-derived fibers are treated with a treatment solution comprising one or more polycarboxylic acids or salts thereof, preferably a polycarboxylic acid as defined in the present disclosure. Optionally, a drying step may be included. Finally, a curing step is required to obtain the absorbent undergarment. At least condition (iv) is fulfilled and preferably all of the following conditions may be fulfilled in the method for manufacturing the absorbent undergarment.
(i) the concentration of the polycarboxylic acid in the treatment solution is 2 to 80 wt.-%, preferably 2.5 to 50 wt.-% and more preferably 10 to 20% wt.-% based on the total weight of the treatment solution,
(ii) the treatment time is 20 sec to 30 min, preferably 30 sec to 15 min, in particular 30 sec to 5 min,
(iii) the curing time is 10 sec to 10 min, preferably 10 sec to 1 min, in particular 10 sec to 30 sec,
(iv) the curing temperature is 140 °C to 200 °C, preferably 140 °C to 180 °C, more preferably 140 °C to 170 °C.

The treatment solution may comprise a catalyst. The catalyst may be selected from the group consisting of sodium hypophosphite, disodium monohydrogen phosphate, sodium dihydrogen phosphate, sodium salt of maleic acid or sodium pyrophosphate.

The selected layer of the absorbent undergarment according to the present disclosure may have a pH after one washing cycle of from 2.5 to 6.5 when measured according to the method described herein. The selected layer of the absorbent undergarment according to the present disclosure may have a pH after 5 washing cycles of from 2.5 to 6.5 when measured according to the method described herein.

Benefits of absorbent undergarments in accordance with the present disclosure will now be described with reference to the following non-limiting examples.

### Example 1: Antibacterial effect

In this example the antibacterial effect of polycarboxylic acid bonded to naturally-derived fibers in cotton and bamboo viscose fabric samples was tested. It was shown that with the presence of citric acid reduces the growth of the tested bacteria.

### Test method for measuring bacterial growth

The antibacterial effect was measured using the standard method AATC100 "Assessment of Antibacterial Finishes on Textile Materials".

Briefly, bacterial strains *Escherichia coli* (ATCC 1175), *Enterococcus faecalis* (P120 Clinical Isolate) and *Staphylococcus epidermidis* (P136 Clinical Isolate) were diluted in 100% Tryptic Soy Broth (TSB) to obtain a start concentration of log 5 colony-forming units per milliliter (10⁵ CFU/ml). Alternatively, a different strain from the species *Escherichia coli, Enterococcus faecalis* or *Staphylococcus epidermidis* may be used in this test method. Test materials were inoculated with 1 ml of respective bacterial cultures and incubated at 35°C for 6 hours. Bacteria were released from the fabric by running the samples for 1 minute at high speed using Letheen Broth in a Stomacher paddle blender (Stomacher 400, Seward). The bacteria were plated on Tryptic Soy Agar (TSA) and incubated for 20h at 35°C until colonies were countable. Bacterial growth was measured as colony-forming units per milliliter (CFU/ml) by plate counting. Number of bacteria were compared to both initial numbers and to the reference material.

### Materials used

The following fabric samples were tested: a knitted two-thread fleece fabric consisting of 100% cotton and a knitted two-thread fleece fabric consisting of 70% bamboo viscose and 30% cotton. The samples were fully emerged in a lukewarm aqueous solution containing 10% Citric Acid, available from Fisher Scientific, and 5% Sodium Hypophosphite, available from Fisher Scientific, for 5 minutes. After removing the excess water, the fabric samples were cured for 10 minutes at 160 degrees Celsius and rinsed in lukewarm water. Next, the fabric samples were machine-washed (WKG130 NDS TDos, Miele) at 60 degrees Celsius using pre-programmed settings optimized for cotton washing in the presence of 50 ml of a two-component liquid washing detergent (UltraPhase I &II, Miele). As a reference material, the same fabric material was used without any kind of treatment.

### Results

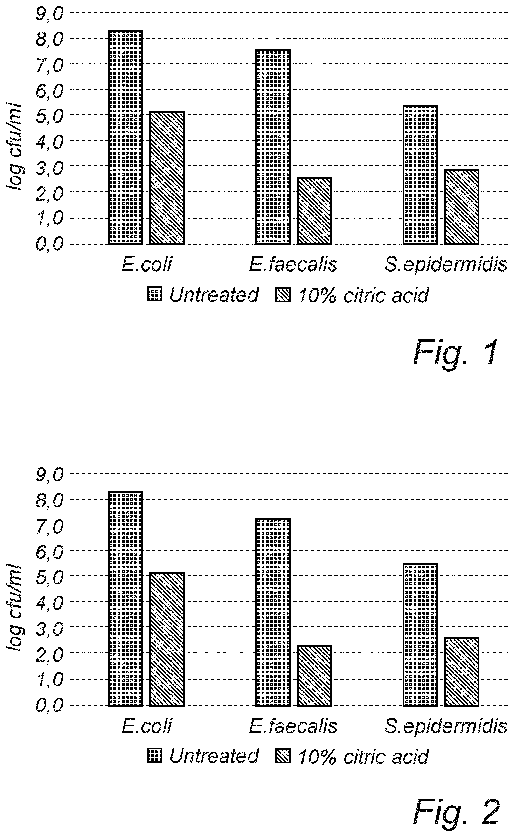

Results from the bacterial growth measurements on the knitted two-thread fleece fabric consisting of 100% cotton are shown in figure 1. Results from the bacterial growth measurement on the knitted two-thread fleece fabric consisting of 70% bamboo viscose and 30% cotton are shown in figure 2.

From these results, it is clear that treatment with Citric Acid reduces bacterial growth on cotton and bamboo viscose fabric samples.

### Example 2: pH stability

In this example pH was measured on treated cotton and bamboo viscose fabric samples after washing. Fabric samples treated with a Citric Acid-solution were compared to untreated samples. It was shown that the treatment with Citric Acid confers a low pH to the fabric sample that remains low after washing.

### Test Method for measuring pH of fabric sample

The pH of the surface of the fabric sample was measured using a pH meter (PH-METER, VWR^{™} SYMPHONY, SB 80PI and the pH electrode used was HAMILTON 10 FLATRODE).

The treated fabric was rinsed in tap water and wringed out. The pH meter rod was rinsed with deionized water and dipped in 0,9% NaCl solution. A small droplet of saline solution was hanging from the electrode and wetted the fabric sample at the point of measurement. All the pH measurements were performed at 23°C and approx.50% RH.

### Materials used

The following fabric samples were tested: a knitted two-thread fleece fabric consisting of 100% cotton, a knitted two-thread fleece fabric consisting of 70% bamboo viscose and 30% cotton and a knitted two-thread fleece fabric consisting of a 55 % hemp/45% cotton blend. The samples were fully emerged in a lukewarm aqueous solution containing 10% Citric Acid, available from Fisher Scientific, and 5% Sodium Hypophosphite, available from Fisher Scientific, for 5 minutes. After removing the excess water, the fabric samples were cured for 10 minutes at 160 degrees Celsius and rinsed in lukewarm water. Next, the fabric samples were machine-washed (WKG130 NDS TDos, Miele) at 60 degrees Celsius using pre-programmed settings optimized for cotton washing in the presence of 50 ml of a two-component liquid washing detergent (UltraPhase I &II, Miele). As a reference material, the same fabric material was used without any kind of treatment.

### Results

Results from the pH measurements are shown below in table 1. Results shown pH measurement after washing of the fabric samples as described above.

| Fabric Sample | pH untreated reference | pH treated sample |
|---|---|---|
| 100% Cotton | 7.2 | 5.6 |
| 70% Bamboo Viscose | 7.0 | 5.2 |
| 55% Hemp 45% Cotton | 7.2 | 5.7 |

From the results above, it is clear that a treatment with citric acid of fabric samples maintains a lower pH after washing as compared to untreated fabric samples.

### Example 3: Effect of curing step and catalyst

In this example, fabric samples were treated with a Citric Acid-solution in the presence or absence of a catalyst and with and without curing. It was shown that the treatment with Citric Acid in the presence of a catalyst and with a curing step confers a low pH to the fabric sample that remains low after washing.

### Test Method for measuring pH of fabric sample

The pH of the surface of the fabric sample was measured using a pH meter (PH-METER, VWR^{™} SYMPHONY, SB 80PI and the pH electrode used was HAMILTON 10 FLATRODE).

The treated fabric was rinsed in tap water, wringed out and followed by dipping in 0,9% NaCl solution before being wringed out again for the pH measurement. The pH meter rod was rinsed with deionized water and dipped in 0,9% NaCl solution and then placed on the fabric sample for measurement. A small droplet of saline solution was hanging from the electrode and wetted the fabric sample at the point of measurement. All the pH measurements were performed at 23°C and approx.50% RH.

### Materials and treatments

In this test cotton fabric samples (100% cotton) were subjected to different treatment conditions according to table 2. The samples were treated with citric acid with or without temperature curing and with and without catalyst. Sodium hypophosphite was used as a catalyst in sample 4 and 6, whereas di-sodium hydrogen phosphate was used as a catalyst in sample 5 and 7. As a reference, the same fabric material was used without any kind of treatment (sample 1). The samples 2 to 7 were fully emerged in an aqueous solution for 5 minutes containing 20% Citric Acid. In the catalyst treated samples the solution also contained 10% Sodium Hypophosphite or 18% di-sodium hydrogen phosphate (both catalysts hence in the same molar concentration). All chemicals used are available from Fisher Scientific. After treatment the excess solution was removed by wringing and left to dry in room temperature or cured for 5 minutes at 155 degrees Celsius.

All samples were laundred in a household washing machine (WKG130 NDS TDos, Miele) at 40 degrees Celsius using pre-programmed settings with standard detergent (Grumme flytande kulör, by Orkla Care).

### Results

Results from the pH measurements are shown below in table 2. Results show pH measurement after washing of the fabric samples as described above.

| | Curing | Catalyst Added | Resulting pH |
|---|---|---|---|
| Sample 1 = reference | No | No | 6,4 |
| Sample 2 | No | No | 6,4 |
| Sample 3 | Yes | No | 5,7 |
| Sample 4 | Yes | Yes | 4,6 |
| Sample 5 | Yes | Yes | 5,4 |
| Sample 6 | No | Yes | 6,3 |
| Sample 7 | No | Yes | 6,5 |

From the results above, it is clear that a treatment with citric acid of fabric samples in the presence of a catalyst and with an additional curing step has a lower pH after washing as compared to fabric samples treated with citric acid in the absence of a catalyst and curing step.

## Claims

1. An absorbent undergarment (1) comprising a fabric layer (2) defining a waist opening (5), two leg openings (6a and 6b) and a crotch region (7) between the leg openings, said absorbent undergarment (1) further comprising an absorbent pad (10) covering at least a part of said crotch region (7) and being permanently attached to the fabric layer (2), said absorbent pad (10) comprising at least one absorbent layer (11) and a skin-facing layer (15), wherein at least one layer, selected from said absorbent layer (11), said skin-facing layer (15) or an optionally present wicking layer (16), comprises naturally-derived fibers comprising a polycarboxylic acid, and wherein the polycarboxylic acid has at least one carboxyl group covalently bound to the naturally-derived fibers, wherein the at least one carboxyl group has reacted with a free hydroxyl group of the naturally-derived fibers, and at least one free carboxyl group.

2. The absorbent undergarment according to claim 1, wherein the polycarboxylic acid has two to ten carboxyl groups, preferably three to five carboxyl groups, and more preferably three carboxyl groups.

3. The absorbent undergarment according to claim 1 or 2 wherein the polycarboxylic acid has 2 to 12 carbon atoms in a main chain, e.g., 4 to 6 carbon atoms in a main chain, wherein the compound is preferably selected from the group consisting of citric acid, isocitric acid, citraconic acid, tartaric acid, itaconic acid, 1,2,3-propanetricarboxylic acid, 1,2,3,4-butanetetracarboxylic acid, glutaric acid, alpha-ketoglutaric acid, malic acid, malonic acid, 2-hydroxymalonic acid, oxalic acid, oxalosuccinic acid, succinic acid, carboxy succinic acid, 1,2 dimethylsuccinic acid, adipic acid, pimelic acid, 2-methyl tricarballylic acid, aconitic acid, 1,2,4-butane tricarboxylic acid, polymers of acrylic acid, and mixtures thereof, wherein the acid is preferably citric acid.

4. The absorbent undergarment according to any one of the claims 1 to 3, wherein at least one carboxyl group is covalently bound to the naturally-derived fibers by forming an ester group with a hydroxy group present in the naturally-derived fiber.

5. The absorbent undergarment according to any of the preceding claims, wherein said at least one layer comprises between 20% and 100% naturally-derived fibers.

6. The absorbent undergarment according to any of the preceding claims, wherein said at least one layer comprising naturally-derived fibers is substantially free of petroleum-based materials.

7. The absorbent undergarment according to any of the claims 1 to 6, wherein the naturally-derived fibers comprising a polycarboxylic acid are obtainable by treating the naturally-derived fibers with a polycarboxylic acid, preferably with a polycarboxylic acid as defined in any of the claims 2 to 3, followed by a curing step.

8. The absorbent undergarment according to claim 7, wherein the treatment of the naturally-derived fibers with a polycarboxylic acid, preferably with a polycarboxylic acid as defined in any of the claims 2 to 3, occurs in the presence of a catalyst.

9. Method for producing an absorbent undergarment as defined in any of claims 1 to 8, the method comprising the steps of:
(i) treating naturally-derived fibers which are present in at least one layer, selected from said absorbent layer (11), said skin-facing layer (15) or an optionally present wicking layer (16), with a treatment solution comprising one or more polycarboxylic acid, or optionally a mixture of one or more polycarboxylic acids or salts thereof, preferably a polycarboxylic acid as defined in any one of claims 2 to 4;
(ii) optionally drying; and
(iii) curing the treated and optionally dried fibers at a temperature of 140°C to 200°C.

10. The method according to claim 9, wherein at least one of the following conditions is fulfilled:
(i) the concentration of the polycarboxylic acid in the treatment solution is 2 to 80 wt.-% based on the total weight of the treatment solution,
(ii) the treatment time is 20 sec to 30 min,
(iii) the curing time is 10 sec to 10 min.

11. The method according to claim 9 or 10, wherein the treatment solution comprises a catalyst.

12. The method according to any one of claims 9 to 11, wherein the absorbent undergarment comprises a fabric layer (2) defining a waist opening (5), two leg openings (6a and 6b) and a crotch region (7) between the leg openings and wherein the absorbent pad (10) covers at least part of the crotch region and is permanently attached to the fabric layer (2).

13. The absorbent undergarment according to claim 8 or the method according to claim 11, wherein the catalyst is selected from the group consisting of sodium hypophosphite, disodium monohydrogen phosphate, sodium dihydrogen phosphate, sodium salt of maleic acid or sodium pyrophosphate.

14. The absorbent undergarment according to any of claims 1 to 8 or 13, wherein said at least one layer comprising naturally-derived fibers has a pH after one washing cycle of from 2.5 to 6.5 when measured according to the method described herein.

15. The absorbent undergarment according to claim 14, wherein said at least one layer comprising naturally-derived fibers has a pH after 5 washing cycles of from 2.5 to 6.5 when measured according to the method described herein.

## Patentansprüche

1. Absorbierendes Unterbekleidungsstück (1) umfassend eine Stofflage (2), die eine Taillenöffnung (5), zwei Beinöffnungen (6a und 6b) und einen Schrittbereich (7) zwischen den Beinöffnungen definiert, wobei das absorbierende Unterbekleidungsstück (1) ferner ein absorbierendes Polster (10) umfasst, das mindestens einen Teil des Schrittbereichs (7) bedeckt und dauerhaft an der Stofflage (2) befestigt ist, wobei das absorbierende Polster (10) mindestens eine absorbierende Schicht (11) und eine der Haut zugewandte Schicht (15) umfasst, wobei mindestens eine Schicht, ausgewählt aus der absorbierenden Schicht (11), der der Haut zugewandten Schicht (15) oder einer optional vorhandenen Dochtschicht (16), natürlich gewonnene Fasern umfasst, die eine Polycarbonsäure umfassen, und wobei die Polycarbonsäure mindestens eine Carboxylgruppe, die kovalent an die natürlich gewonnenen Fasern gebunden ist, wobei die mindestens eine Carboxylgruppe mit einer freien Hydroxylgruppe der natürlich gewonnenen Fasern umgesetzt wurde, und mindestens eine freie Carboxylgruppe aufweist.

2. Absorbierendes Unterbekleidungsstück gemäß Anspruch 1, wobei die Polycarbonsäure zwei bis zehn Carboxylgruppen, vorzugsweise drei bis fünf Carboxylgruppen und noch bevorzugter drei Carboxylgruppen aufweist.

3. Absorbierendes Unterbekleidungsstück gemäß Anspruch 1 oder 2, wobei die Polycarbonsäure 2 bis 12 Kohlenstoffatome in einer Hauptkette aufweist, z. B. 4 bis 6 Kohlenstoffatome in einer Hauptkette, wobei die Verbindung vorzugsweise aus der Gruppe bestehend aus Zitronensäure, Isocitronensäure, Citraconsäure, Weinsäure, Itaconsäure, 1,2,3-Propantricarbonsäure, 1,2,3,4-Butantetracarbonsäure, Glutarsäure, Alpha-Ketoglutarsäure, Apfelsäure, Malonsäure, 2-Hydroxymalonsäure, Oxalsäure, Oxalbernsteinsäure, Bernsteinsäure, Carboxybernsteinsäure, 1,2-Dimethylbernsteinsäure, Adipinsäure, Pimelinsäure, 2-Methyltricarballylsäure, Aconitsäure, 1,2,4-Butantricarbonsäure, Polymere von Acrylsäure und Mischungen davon ausgewählt ist, wobei die Säure vorzugsweise Zitronensäure ist.

4. Absorbierendes Unterbekleidungsstück gemäß mindestens einem der Ansprüche 1 bis 3, wobei mindestens eine Carboxylgruppe durch Bildung einer Estergruppe mit einer in den natürlich gewonnenen Fasern vorhandenen Hydroxygruppe kovalent an die natürlich gewonnenen Fasern gebunden ist.

5. Absorbierendes Unterbekleidungsstück gemäß mindestens einem der vorhergehenden Ansprüche, wobei die mindestens eine Schicht zwischen 20% und 100% natürlich gewonnene Fasern umfasst.

6. Absorbierendes Unterbekleidungsstück gemäß mindestens einem der vorhergehenden Ansprüche, wobei die mindestens eine Schicht, die natürlich gewonnene Fasern umfasst, im Wesentlichen frei von Materialien auf Erdölbasis ist.

7. Absorbierendes Unterbekleidungsstück gemäß mindestsens einem der Ansprüche 1 bis 6, wobei die natürlich gewonnenen Fasern, die eine Polycarbonsäure umfassen, durch Behandeln der natürlich gewonnenen Fasern mit einer Polycarbonsäure, vorzugsweise mit einer in mindestens einem der Ansprüche 2 bis 3 definierten Polycarbonsäure, und anschließendem Aushärten erhältlich sind.

8. Absorbierendes Unterbekleidungsstück gemäß Anspruch 7, wobei die Behandlung der natürlich gewonnenen Fasern mit einer Polycarbonsäure, vorzugsweise mit einer in mindestens einem der Ansprüche 2 bis 3 definierten Polycarbonsäure, in Gegenwart eines Katalysators erfolgt.

9. Verfahren zur Herstellung eines absorbierenden Unterbekleidungsstück gemäß mindestens einem der Ansprüche 1 bis 8, wobei das Verfahren die folgenden Schritte umfasst:
(i) Behandeln von natürlich gewonnenen Fasern, die in mindestens einer Schicht vorhanden sind, ausgewählt aus der absorbierenden Schicht (11), der der Haut zugewandten Schicht (15) oder einer optional vorhandenen Dochtschicht (16), mit einer Behandlungslösung, die eine oder mehrere Polycarbonsäuren oder optional eine Mischung aus einer oder mehreren Polycarbonsäuren oder deren Salzen, vorzugsweise eine in mindestens einem der Ansprüche 2 bis 4 definierten Polycarbonsäure, umfasst;
(ii) optionales Trocknen; und
(iii) Aushärten der behandelten und optional getrockneten Fasern bei einer Temperatur von 140°C bis 200°C.

10. Verfahren gemäß Anspruch 9, bei dem mindestens eine der folgenden Bedingungen erfüllt ist:
(i) die Konzentration der Polycarbonsäure in der Behandlungslösung beträgt 2 bis 80 Gew.-%, bezogen auf das Gesamtgewicht der Behandlungslösung,
(ii) die Behandlungszeit beträgt 20 Sekunden bis 30 Minuten,
(iii) die Aushärtungszeit beträgt 10 Sekunden bis 10 Minuten.

11. Verfahren gemäß Anspruch 9 oder 10, bei dem die Behandlungslösung einen Katalysator umfasst.

12. Verfahren gemäß mindestens einem der Ansprüche 9 bis 11, wobei das absorbierende Unterbekleidungsstück eine Stofflage (2) umfasst, die eine Taillenöffnung (5), zwei Beinöffnungen (6a und 6b) und einen Schrittbereich (7) zwischen den Beinöffnungen definiert, und wobei das absorbierende Polster (10) mindestens einen Teil des Schrittbereichs bedeckt und dauerhaft an der Stofflage (2) befestigt ist.

13. Absorbierendes Unterbekleidungsstück gemäß Anspruch 8 oder Verfahren gemäß Anspruch 11, wobei der Katalysator ausgewählt ist aus der Gruppe bestehend aus Natriumhypophosphit, Dinatriummonohydrogenphosphat, Natriumdihydrogenphosphat, Natriumsalz der Maleinsäure oder Natriumpyrophosphat.

14. Absorbierendes Unterbekleidungsstück gemäß mindestens einem der Ansprüche 1 bis 8 oder 13, wobei die mindestens eine Schicht, die natürlich gewonnene Fasern umfasst, nach einem Waschzyklus einen pH-Wert von 2,5 bis 6,5 aufweist, gemessen gemäß dem hierin beschriebenen Verfahren.

15. Absorbierendes Unterbekleidungsstück gemäß Anspruch 14, wobei die mindestens eine Schicht, die natürlich gewonnene Fasern umfasst, nach 5 Waschzyklen einen pH-Wert von 2,5 bis 6,5 aufweist, gemessen gemäß dem hierin beschriebenen Verfahren.

## Revendications

1. Sous-vêtement absorbant (1) comprenant une couche de tissu (2) définissant une ouverture à la taille (5), deux ouvertures pour les jambes (6a et 6b) et une zone d'entrejambe (7) entre les ouvertures pour les jambes, ledit sous-vêtement absorbant (1) comprenant en outre un coussin absorbant (10) recouvrant au moins une partie de ladite zone d'entrejambe (7) et étant fixé de manière permanente à la couche de tissu (2), ledit coussin absorbant (10) comprenant au moins une couche absorbante (11) et une couche en contact avec la peau (15), dans lequel au moins une couche, choisie parmi ladite couche absorbante (11), ladite couche en contact avec la peau (15) ou une couche de mèche (16) éventuellement présente, comprend des fibres d'origine naturelle comprenant un acide polycarboxylique, et dans lequel l'acide polycarboxylique comporte au moins un groupe carboxyle lié de manière covalente aux fibres d'origine naturelle, dans lequel le au moins un groupe carboxyle a réagi avec un groupe hydroxyle libre des fibres d'origine naturelle, et au moins un groupe carboxyle libre.

2. Le sous-vêtement absorbant selon la revendication 1, dans lequel l'acide polycarboxylique a de deux à dix groupes carboxyle, préférablement de trois à cinq groupes carboxyle, et plus préférablement trois groupes carboxyle.

3. Le sous-vêtement absorbant selon la revendication 1 ou 2, dans lequel l'acide polycarboxylique a de 2 à 12 atomes de carbone dans une chaîne principale, par exemple de 4 à 6 atomes de carbone dans une chaîne principale, dans lequel le composé est préférablement choisi dans le groupe constitué par l'acide citrique, l'acide isocitrique, l'acide citraconique, l'acide tartrique, l'acide itaconique, l'acide 1,2,3-propanetricarboxylique, l'acide 1,2,3,4-butanetétracarboxylique, l'acide glutarique, l'acide alpha-cétoglutarique, l'acide malique, l'acide malonique, l'acide 2-hydroxymalonique, l'acide oxalique, l'acide oxalosuccinique, l'acide succinique, l'acide carboxysuccinique, l'acide 1,2-diméthylsuccinique, l'acide adipique, l'acide pimélique, l'acide 2-méthyl tricarballylique, l'acide aconitique, l'acide 1,2,4-butane tricarboxylique, les polymères d'acide acrylique, et leurs mélanges, dans lesquels l'acide est de préférence l'acide citrique.

4. Le sous-vêtement absorbant selon l'une quelconque des revendications 1 à 3, dans lequel au moins un groupe carboxyle est lié de manière covalente aux fibres d'origine naturelle en formant un groupe ester avec un groupe hydroxy présent dans la fibre d'origine naturelle.

5. Le sous-vêtement absorbant selon l'une quelconque des revendications précédentes, dans lequel ladite au moins une couche comprend entre 20 % et 100 % de fibres d'origine naturelle.

6. Le sous-vêtement absorbant selon l'une quelconque des revendications précédentes, dans lequel ladite au moins une couche comprenant des fibres d'origine naturelle est sensiblement exempte de matériaux à base de pétrole.

7. Le sous-vêtement absorbant selon l'une quelconque des revendications 1 à 6, dans lequel les fibres d'origine naturelle comprenant un acide polycarboxylique peuvent être obtenues en traitant les fibres d'origine naturelle avec un acide polycarboxylique, de préférence avec un acide polycarboxylique tel que défini dans l'une quelconque des revendications 2 à 3, suivi d'une étape de durcissement.

8. Le sous-vêtement absorbant selon la revendication 7, dans lequel le traitement des fibres d'origine naturelle avec un acide polycarboxylique, préférablement avec un acide polycarboxylique tel que défini dans l'une quelconque des revendications 2 à 3, se produit en présence d'un catalyseur.

9. Procédé de fabrication d'un sous-vêtement absorbant tel que défini dans l'une quelconque des revendications 1 à 8, le procédé comprenant les étapes suivantes :
(i) traitement des fibres d'origine naturelle qui sont présentes dans au moins une couche, choisie parmi ladite couche absorbante (11), ladite couche en contact avec la peau (15) ou une couche de mèche (16) optionnellement présente, avec une solution de traitement comprenant un ou plusieurs acides polycarboxyliques, ou optionnellement un mélange d'un ou plusieurs acides polycarboxyliques ou de leurs sels, préférablement un acide polycarboxylique tel que défini dans l'une quelconque des revendications 2 à 4 ;
(ii) optionnellement séchage ; et
(iii) durcissement des fibres traitées et optionnellement séchage à une température de 140 °C à 200 °C.

10. Le procédé selon la revendication 9, dans lequel au moins l'une des conditions suivantes est remplie :
(i) la concentration de l'acide polycarboxylique dans la solution de traitement est de 2 à 80 % en poids par rapport au poids total de la solution de traitement,
(ii) la durée du traitement est de 20 secondes à 30 minutes,
(iii) la durée de durcissement est de 10 secondes à 10 minutes.

11. Le procédé selon la revendication 9 ou 10, dans lequel la solution de traitement comprend un catalyseur.

12. Le procédé selon l'une quelconque des revendications 9 à 11, dans lequel le sous-vêtement absorbant comprend une couche de tissu (2) définissant une ouverture à la taille (5), deux ouvertures pour les jambes (6a et 6b) et une région d'entrejambe (7) entre les ouvertures pour les jambes, et dans lequel le coussin absorbant (10) recouvre au moins une partie de la région d'entrejambe et est fixé de manière permanente à la couche de tissu (2) .

13. Le sous-vêtement absorbant selon la revendication 8 ou procédé selon la revendication 11, dans lequel le catalyseur est choisi dans le groupe constitué par l'hypophosphite de sodium, le monohydrogénophosphate disodique, le dihydrogénophosphate de sodium, le sel sodique de l'acide maléique ou le pyrophosphate de sodium.

14. Le sous-vêtement absorbant selon l'une quelconque des revendications 1 à 8 ou 13, dans lequel ladite au moins une couche comprenant des fibres d'origine naturelle a un pH après un cycle de lavage de 2,5 à 6,5 lorsqu'il est mesuré selon la méthode décrite ici.

15. Le sous-vêtement absorbant selon la revendication 14, dans lequel ladite au moins une couche comprenant des fibres d'origine naturelle a un pH après 5 cycles de lavage de 2,5 à 6,5 lorsqu'il est mesuré selon la méthode décrite ici.
